(19) Europäisches Patentamt
European Patent Office
Office européen des brevets

(11) **EP 2 893 894 B1**

(12) **EUROPEAN PATENT SPECIFICATION**

(45) Date of publication and mention
of the grant of the patent:
**09.12.2020 Bulletin 2020/50**

(51) Int Cl.:
**A61B 18/14** *(2006.01)*    **A61N 1/32** *(2006.01)*
**A61B 17/94** *(2006.01)*    **A61M 5/162** *(2006.01)*
**A61B 18/00** *(2006.01)*    **A61B 1/00** *(2006.01)*

(21) Application number: **13833078.2**

(22) Date of filing: **30.08.2013**

(86) International application number:
**PCT/KR2013/007803**

(87) International publication number:
**WO 2014/035176 (06.03.2014 Gazette 2014/10)**

(54) **ENDOSURGICAL OPERATING DEVICE AND ENDOSCOPIC DEVICE COMPRISING SAME**

ENDOCHIRURGISCHE BETRIEBSVORRICHTUNG UND ENDOSKOPVORRICHTUNG DAMIT

DISPOSITIF D'OPÉRATION ENDOCHIRURGICALE ET DISPOSITIF ENDOSCOPIQUE LE
COMPRENANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **03.09.2012 KR 20120097154**

(43) Date of publication of application:
**15.07.2015 Bulletin 2015/29**

(73) Proprietor: **Na, Jongju**
**Seoul 138-050 (KR)**

(72) Inventor: **Na, Jongju**
**Seoul 138-050 (KR)**

(74) Representative: **Walaski, Jan Filip et al**
**Venner Shipley LLP**
**200 Aldersgate**
**London EC1A 4HD (GB)**

(56) References cited:
WO-A1-2010/022275    WO-A2-2008/005477
JP-A- 2002 514 097    KR-A- 20090 078 306
KR-A- 20090 131 724    US-A- 3 640 270
US-A1- 2002 120 260    US-A1- 2005 107 829
US-A1- 2005 283 147    US-A1- 2006 058 723
US-A1- 2010 240 995    US-A1- 2012 158 100

**Description**

[Technical Field]

The present invention relates to an endosurgical operating device and an endoscopic device including the same, and more specifically to an endosurgical operating device which is provided with an endothelium adsorption means for adherence to an epithelial layer of endothelium, so that a plurality of needles can easily reach a lower tissue of the epithelial layer whereby the treatment effect by the plurality of needles can be sufficiently obtained, and an endoscopic device including the same.

[Background Art]

Internal and external surfaces of a human body are covered with an integument, endothelium and mucous membrane. The integument refers to appendages of a skin such as the skin of the body surface and the sweat glands, and modified substances such as furs or fingernails and toenails.

The endothelium refers to a tissue that covers a closed lumen wall without communicating with an outside of the body, and the mucous membrane refers to a tissue that forms an inner wall of respiratory, digestive and urinogenital organs which come into direct contact with the outside.

In the Oriental medical field, the endothelium and mucous membrane as described above are commonly called an endothelium. Therefore, in the present disclosure, all except the integument will be commonly referred to as the endothelium.

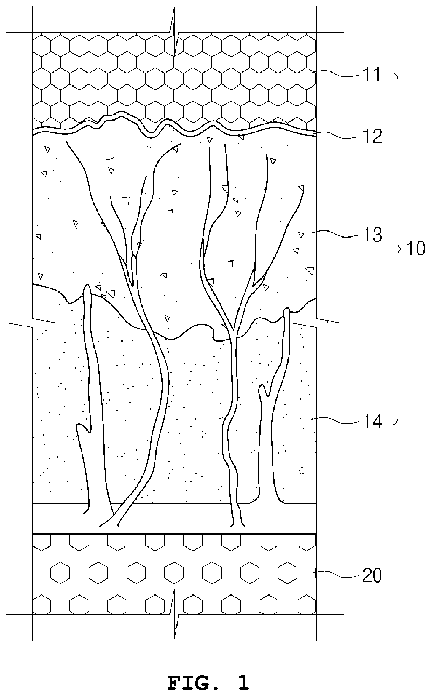

FIG. 1 illustrates a structure of the endothelium. The endothelium illustrated in FIG. 1 is an oral mucosa. The endothelium 10 includes an epithelial layer 11, a basal membrane 12, a lamina propria 13 and a submucosa 14. Bones or muscles 20 may be located below the endothelium 10.

The lamina propria 13 includes collagens. It is a well-known fact that, when the endothelium 10 is damaged, if collagen is allowed to be denatured by transmitting heat energy or electric energy to the lamina propria 13, regeneration of the damaged endothelium is facilitated. There have been various attempts to treat the damaged endothelium by using the above-described fact.

For example, there are techniques disclosed in Korean Patent Laid-Open Publication Nos. 2006-0088504 and 2009-0131724. The former discloses a technique in which a hemostatic effect may be obtained by applying electric energy at a medical radio-frequency band to a styptic injection needle provided in an endoscope, and the latter discloses a technique in which an affected area may be treated, for example, by destroying tumor bacteria, by allowing heat to be transmitted to the endothelium using an acupuncture needle provided in the endoscope.

However, when the above-described injection needle or acupuncture needle is applied to the endothelium, which is a very soft tissue, applying force in order for the injection needle or acupuncture needle to reach the lamina propria 13 causes a problem that the epithelial layer 11 is deformed without being penetrated or sufficiently penetrated, so that the injection needle or acupuncture needle does not reach the lamina propria 13.

If the injection needle or acupuncture needle cannot reach a proper depth, the intended treatment effect cannot be obtained as heat energy or electric energy applied through the needle cannot be sufficiently transmitted to the lamina propria 13, or the energy that could not be transmitted to the lamina propria 13 acts on the epithelial layer 11, so as to bring about a side effect such as causing a burn to the epithelial layer 11.

Further, if the thickness of the epithelial layer 11 is very thin, there is a high possibility that energy transmitted through the needle gives an impact to the epithelial layer 11, even if the injection needle or acupuncture needle sufficiently reaches the lamina propria 13.

Further, when a small number of needles such as one or a pair of needles like the above-described injection needle or acupuncture needle are used, the operation should be repeated many times if the area to be treated is wide. Therefore, much time and effort may be needed to treat the endothelium.

Further, since there is a high possibility that the endothelium 10 will bleed through the portion penetrated by the injection needle or acupuncture needle, countermeasures are needed so as to suppress bleeding as much as possible and minimize infection due to leaked blood.

US2002120260, US2005107829, WO2010022275, US3640270 and WO2008005477 refer to prior art documents relevant for the present invention. US2002120260 is considered to be the closest prior art.

[Disclosure]

[Technical Problem]

Therefore, it is an object of the present invention to provide an endosurgical operating device which is provided with an endothelium adsorption means for adherence to an epithelial layer of endothelium, so that a plurality of needles can easily reach a lower tissue of the epithelial layer whereby the treatment effect by the plurality of needles can be sufficiently obtained, and an endoscopic device including the same.

[Technical Solution]

The present invention is defined by the appended independent claim 1. Preferred embodiments are disclosed in the dependent claims.

In order to accomplish the above object, according to one aspect of the present invention, there is provided an endosurgical operating device including: an operation tube 111 which is installed in an insertion part 5 of an endoscope 1 to be inserted in a body; an operation plate 122 which is installed in the operation tube 111 and includes at least one needle 121 provided with a pointed tip; a driving unit 165 configured to drive the operation plate 122 so that at least one of the needles passes through an end portion of the operation tube 111, and is inserted into an internal tissue surface or an epithelium surface in the body; and a power supply unit 140 which is electrically connected to the at least one needle 121.

According to another aspect, there is provided an endosurgical operating device including: an operation tube 111 which is installed in an insertion part 5 of an endoscope 1 to be inserted into a body; an operation plate 122 which is installed in the operation tube 111 and includes at least one protrusion 800 provided with a pointed tip; a driving unit 165 configured to drive the operation plate 122 so that the pointed tip passes through an end portion of the operation tube 111, and is moved to an internal tissue surface or an epithelium surface in the body; and a power supply unit 140 which is electrically connected to the protrusion 800.

The endosurgical operating device further includes an adsorption means 170 which is provided in the end portion of the operation tube 111, and adheres to the internal tissue surface or the epithelium surface in the body in an adsorption method.

In addition, at least one of the adsorption means 170 may include an insulation member.

Further, the adsorption means 170 has at least one adsorption hole formed therein, and the driving unit 165 drives the operation plate 122 so that the pointed tip passes through the end portion of the operation tube 111 through the at least one adsorption hole.

Further, the adsorption means 170 is configured to absorb substances discharged from the tissue surface or the epithelium surface into the endosurgical operating device through the adsorption hole, when the at least one needle 121 is inserted into the internal tissue surface or the epithelium surface in the body.

Further, the endosurgical operating device may further include a temperature sensor configured to detect a temperature of the adsorption means 170.

Further, the at least one needle 121 may have a hollow portion formed therein, and include a drug container connected to the hollow portion.

Further, the endosurgical operating device may further include a surface temperature control means 499 configured to control the temperature of an operation portion in contact with the tissue surface or the epithelium surface.

Further, the pointed tip may be configured to receive a high-frequency current applied thereto, and the pointed tip may have a bipolar electrode system.

Further, at least one of the pointed tips may have a polarity alternating with the polarity of a neighboring pointed tip.

Further, the endosurgical operating device may further include a control unit 151 configured to control the driving unit 165 so that the at least one needle is inserted into the tissue surface or the epithelium surface to a preset depth.

In addition, according to another aspect, there is provided an endoscopic device including: the above-described endosurgical operating device.

[Advantageous Effects]

According to the present invention, the endosurgical operating device is provided with an endothelium adsorption means for adherence to the epithelial layer of endothelium, so that treatment effects of the endothelium may be sufficiently obtained by allowing the plurality of needles to easily reach the lower tissue of the epithelial layer.

In addition, according to the present invention, it is possible to minimize the treatment effect for the lamina propria affecting the outer layer of the skin or maximize the treatment effects, by regulating the temperature of the outer layer of the endothelium.

Further, the endosurgical operating device is provided with the plurality of needles distributed so as to have a predetermined area, it is possible to treat the whole of the affected area with a small number of operations even in the case of a wide affected area.

Further, according to the present invention, by making it possible to regulate the temperature of the portion in contact with the endothelium, it is possible to minimize bleeding that could occur as the endothelium is penetrated, or facilitate the recovery of the affected area by regulating the temperature of the endothelium to the maximum activation temperature of the drug that facilitates the recovery of the damaged endothelium.

Further, according to the present invention, if bleeding occurs, it is possible to minimize the risk of blood-mediated infection by preventing blood from contacting the surgical space or operator.

[Description of Drawings]

FIG. 1 is a cross-sectional view illustrating a structure of an endothelium.

FIG. 2 is a schematic view illustrating an endoscope includes an endosurgical operating device according to a first embodiment of the present invention.

FIG. 3 is an enlarged longitudinal sectional view of portion A illustrated in FIG. 1.

FIG. 4 is a cross-sectional view for describing an operation of the endosurgical operating device according to the first embodiment of the present invention.

FIG. 5 is a block diagram schematically illustrating a configuration of the endosurgical operating device according to the first embodiment of the present invention.

FIG. 6 is a bottom view of the endosurgical operating device according to the first embodiment of the present invention.

FIGS. 7 to 9 are enlarged views of portion B illustrated in FIG. 3 for describing the operation of the endosurgical operating device.

FIG. 10 is an enlarged view of portion C illustrated in FIG. 9.

FIG. 11 is a longitudinal sectional view of the endosurgical operating device according to a second embodiment not forming part of the present invention.

FIG. 12 is a longitudinal sectional view of an endothelium adsorption means illustrated in FIG. 11.

FIGS. 13 and 14 are enlarged views illustrating modified examples of the adsorption portion of the endothelium adsorption means.

FIG. 15 is a longitudinal sectional view illustrating a modified example of the endothelium adsorption means.

FIG. 16 is a longitudinal sectional view of a tip portion of the endosurgical operating device according a third embodiment not forming part of the present invention.

FIG. 17 is an enlarged view illustrating an adsorption part of the endosurgical operating device according to a fourth embodiment not forming part of the present invention.

FIG. 18 is an enlarged view illustrating an adsorption part of the endosurgical operating device according to a fifth embodiment not forming part of the present invention.

FIG. 19 is a cross-sectional view illustrating surgical results in which Na-effect is achieved in an endosurgical operating device according to a sixth embodiment of the present invention.

[Best Mode]

Hereinafter, the present invention will be described with reference to the accompanying drawings 1-10 and 19 in detail. Referring to the drawings, wherein like reference characters designate like or corresponding parts throughout the several views. In the embodiments of the present invention, the publicly known functions and configurations that are considered to be able to make the purport of the present invention unnecessarily obscure will not be described.

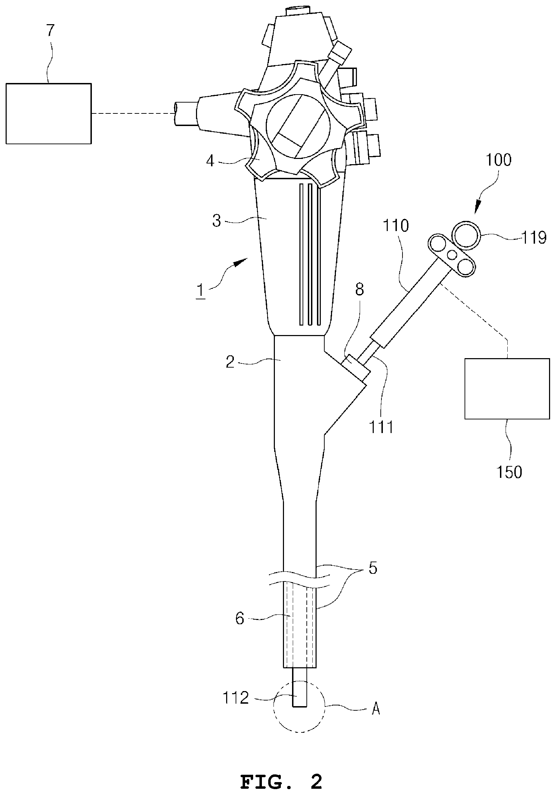

FIG. 2 is a schematic view illustrating an endoscope provided with an endosurgical operating device according to a first embodiment of the present invention.

Referring to FIG. 2, an endoscope 1 provided with an endosurgical operating device according to the first embodiment of the present invention includes an endosurgical operating device 100. The endoscope 1 includes a body part 2, a handle part 3, an operation part 4, an insertion part 5 and a supply unit 7.

Meanwhile, the endosurgical operating device 100 may include a treatment device body 110, an operation tube 111 such as a sheath, etc., and an operation handle 119. The operation tube 111 is connected to one end of the endosurgical operating device 100, and the operation handle 119 may be provided on the other end of the endosurgical operating device 100.

The body part 2 is provided with the handle part 3 and the operation part 4, and the insertion part 5 is connected to one end thereof. Herein, the insertion part 5 is a portion whose other end is inserted into a body cavity, and may be manufactured so as to have flexibility according to the use thereof.

The supply unit 7 connected to the body part 2 is a means for supplying a fluid such as air or water, electric power, etc., which are necessary for surgery using the endoscope 1.

The handle part 3 is a portion gripped by an operator executing surgery using the endoscope 1, and may be provided with the operation part 4. The operation part 4 can make the insertion part 5 bend in a direction required by the operator, make air or water supplied from the supply unit 7 be discharged through an end portion of the insertion part 5, or control the operations of a lighting means (not illustrated) or a imaging means (not illustrated) which are provided on the other end of the insertion part 5.

One or more channel 6 may be formed in the insertion part 5 in a lengthwise direction thereof. The channel 6 may be used as a passage for supplying fluids supplied from the supply unit 7 or sucking blood or body fluids around the other end of the insertion part 5 into the supply unit 7, and may also be utilized as a path in which tools necessary for surgery using the endoscope 1 are moved.

The body part 2 has an insertion port 8 formed therein to communicate with the channel 6. The operation tube 111 may be inserted into the channel 6 through the insertion port 8. At this time, the operator can make the other end portion of the operation tube 111, that is, the front end part 112 of the operation tube, protrude in a predetermined length from the end portion of the insertion part 5, by controlling a length in which the operation tube 111 is inserted into the insertion port 8, using the operation handle 119.

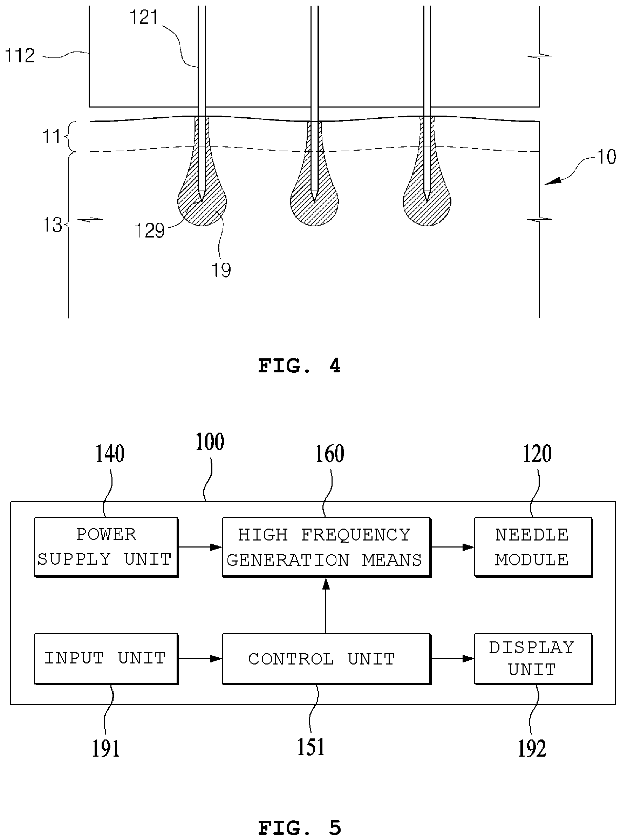

A control and supply unit 150 connected to the treatment device body 110 plays a role of supplying electric power necessary for treatment of the endothelium using the treatment device body 110. The control and supply unit 150 will be described below again with reference to FIG. 5.

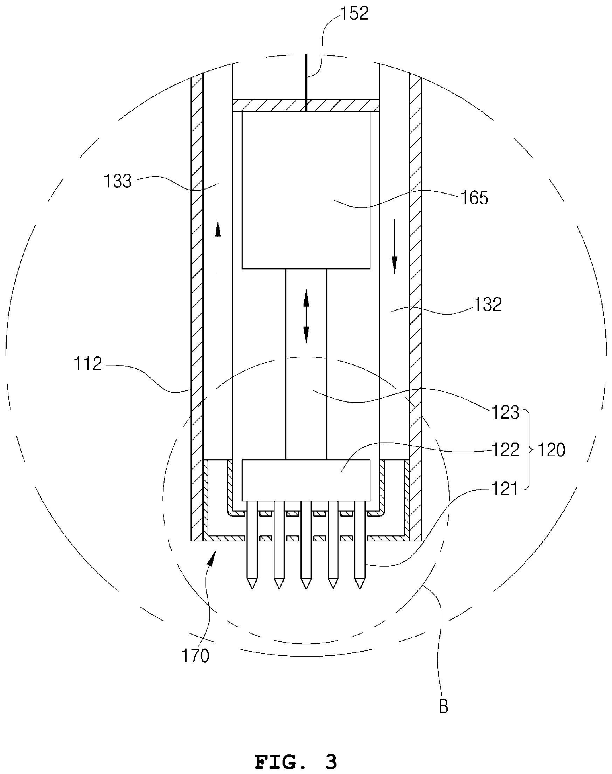

FIG. 3 is an enlarged longitudinal sectional view of portion A illustrated in FIG. 1.

Referring to FIG. 3, the endosurgical operating device 100 may include a needle module 120, an air supply tube 132, an air exhaust tube 133, a power transmission member 152, a driving unit 165 and an endothelium adsorption means 170, and a temperature sensor (not illustrated).

The operation tube front end part 112 may have an endothelium adsorption means 170 installed therein as illustrated in FIG. 3. The endothelium adsorption means 170 is installed such that a part thereof may be exposed out of the operation tube front end part 112. That is, the endothelium adsorption means 170 may be disposed in a shape coupled to the other end of the operation tube 111.

The needle module 120 may include a plurality of needles 121, an operation plate 122 and a connection member 123. The plurality of needles 121 respectively have pointed tips. The plurality of needles 121 are coupled to the operation plate 122 dispersedly in parallel. Meanwhile, it is preferable that the pointed tips of the plurality of needles 121 are sharply and pointedly formed so as to be inserted through a tissue surface or an endothelium surface in a human body.

At this time, the plurality of needles 121 are disposed in parallel so that all the pointed tips are oriented in one direction. That is, as illustrated in FIG. 2, the pointed tips are disposed so as to face the outward direction of the operation tube front end part 112.

The connection member 123 is coupled to and/or formed on a portion of the operation plate 122 opposite to the portion to which the plurality of needles 121 are coupled.

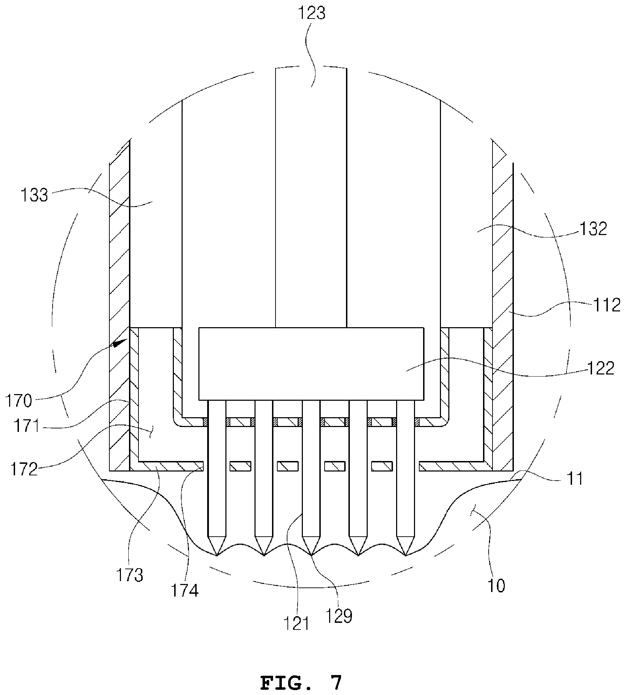

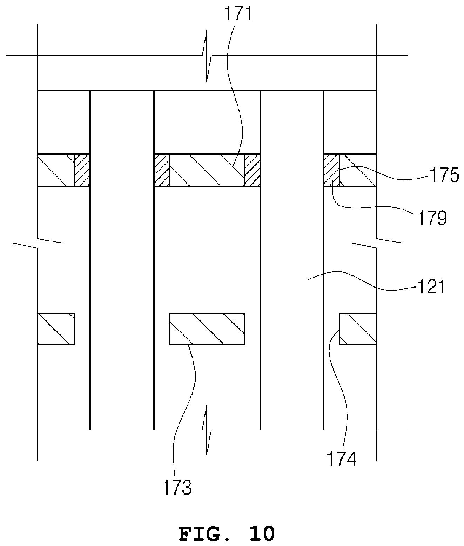

The needle module 120 may be disposed in the other end of the operation tube 111 of FIG. 2, that is, in the operation tube front end part 112. At this time, the needle module 120 is disposed in a shape in which the plurality of needles 121 penetrate the endothelium adsorption means 170. That is, the endothelium adsorption means 170 have a plurality of penetration holes 174 and 175 of FIG. 10 which are formed therein and described below. The operation plate 122 is disposed inside of the operation tube front end part 112 from the endothelium adsorption means 170, and the plurality of needles 121 are disposed outside of the other end portion of the operation tube 111 through the penetration holes 174 and 175, that is, in a shape protruding in the outward direction of the operation tube front end part 112, which will be described in more detail with reference to FIG. 7.

FIG. 7 is an enlarged longitudinal sectional view of portion A illustrated in FIG. 3.

Referring to FIG. 7, the endothelium adsorption means 170 includes an adsorption means body 171. An air flow space 172 and the plurality of penetration holes 174 are formed in the adsorption means body 171. In addition, a plate-shaped adsorption plate portion 173 is formed in the adsorption means body 171. The adsorption plate portion 173 is exposed outward of the operation tube front end part 112 and comes in contact with the endothelium 10.

One hole(A portion) 174 of the plurality of penetration holes 174 and 175 of FIG. 10 is disposed in the adsorption plate portion 173, and the other hole 175 is disposed in a position facing the operation plate 122 in the adsorption means body 171. The plurality of penetration holes 174 and 175 are formed so as to correspond to the position and number of the plurality of needles 121, so that the plurality of needles 121 can penetrate the plurality of penetration holes 174 and 175 respectively as illustrated in FIG. 10.

In addition, as illustrated in the drawings, the plurality of penetration holes 174 and 175 may be formed so as to communicate with the air flow space 172.

Referring to FIG. 3 again, the driving unit 165 may be disposed in the other end of the operation tube 111, that is, in the operation tube front end part 112 as illustrated therein.

The driving unit 165 can drive the connection member 123 in the lengthwise direction of the connection member 123, that is, the direction from one end to the other end of the operation tube 111, or the direction parallel with the lengthwise direction of the plurality of needles 121.

When driving the connection member 123, the driving force thereof is transmitted to the plurality of needles 121 through the operation plate 122. Therefore, it may be driven in such a manner that the plurality of needles 121 protrude outward of the operation tube front end part 112 through the penetration hole 174 or retreated inward of the penetration hole 174.

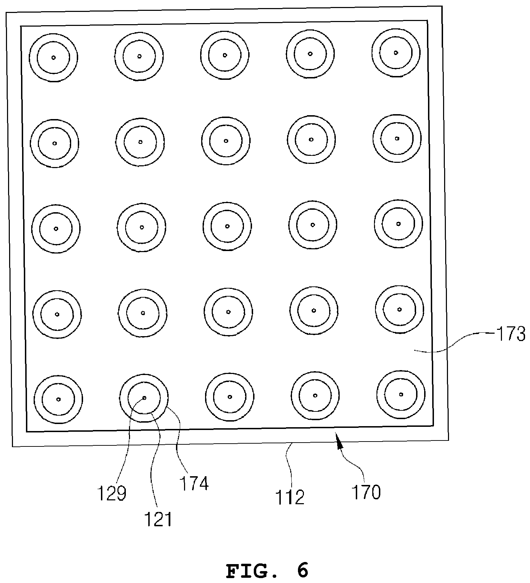

Therefore, the driving unit 165 can make the adsorption plate portion 173 of FIG. 6 contact the endothelium 10, and then drive such that the plurality of needles 121 protrude in the direction of one end of the operation tube 111 so as to be inserted into the endothelium 10, or make the plurality of needles 121 retreat in the opposite direction so that the plurality of needles 121 that were inserted into the endothelium 10 come off the endothelium 10.

Alternately, it is also possible that the plurality of needles 121 protruded outward of the operation tube front end part 112 are inserted into the endothelium 10, by making the operation tube 111 move in the direction of the operation tube front end part 112 using the operation handle 119 provided in the treatment device body 110.

For reference, a variety of means for the connection member 123 to make reciprocal linear motion such as a motor, a linear motor, an electromagnet and a piezoelectric sensor may be used as the driving unit 165.

Although not illustrated in detail, the power transmission member 152 may electrically connect a high-frequency generation means 160 of FIG. 5, which will be described below, and the needle 121 each other so that high-frequency electric energy generated from the high-frequency generation means 160 is transmitted to the needle 121.

For reference, the high-frequency generation means 160 generates electricity by using various elements such as coils, vacuum tubes and transistors. Since devices outputting high-frequency electric energy with electric power supplied are well known to those skilled in the art, they will not be described.

Although not illustrated in the drawing, the high-frequency generation means 160 may be disposed in the operation tube 111, in the operation tube front end part 112, in the treatment device body 110, or the like.

One end of the air supply tube 132 is separately connected to the endothelium adsorption means 170, and the other end thereof may be extended to the outside of the operation tube 111. At this time, one end of the air supply tube 132 is connected so as to communicate with the air flow space 172 of FIG. 7 formed in the endothelium adsorption means 170.

For reference, the operation tube front end part 112 may be configured separately from the operation tube 111. This is to make it easy to replace the components embedded in the other end of the operation tube 111 or in the operation tube front end part 112.

For instance, if the operation tube front end part 112 is separated from the operation tube 111, the needle module 120 and the endothelium adsorption means 170 may be separated from the operation tube 111, and thereby the air supply tube 132 may be separated from the endothelium adsorption means 170.

One end of the air exhaust tube 133 is detachably connected to the endothelium adsorption means 170. At this time, the portion, to which the air exhaust tube 133 of the endothelium adsorption means 170 is connected, may be configured such that air introduced into the air flow space 172 of FIG. 7 through the air supply tube 132 flows to the air exhaust tube 133 after uniformly circulated in the air flow space 172 of FIG. 7. That is, the air supply tube 132 and the air exhaust tube 133 may be respectively connected to the endothelium adsorption means 170 at positions in symmetry with each other. For reference, if the operation tube front end part 112 is separated from the operation tube 111 as described above, the air exhaust tube 133 also may be separated from the endothelium adsorption means 170.

An air flow means (not illustrated) may be installed on the other end of the air exhaust tube 133. This will be described with reference to FIG. 5.

FIG. 5 is a schematic view illustrating the configuration of an endosurgical operating device according to the first embodiment of the present invention.

Referring to FIG. 5, the endosurgical operating device 100 according to the first embodiment of the present invention may further include a power supply unit 140, a control unit 151, an input unit 191, a display unit 192, an air flow means (not illustrated) and a temperature control means (not illustrated) in addition to the needle module 120 and the high-frequency generation means 160.

The control unit 151 may be installed so as to be electrically connected to the temperature control means (not illustrated), the air flow means (not illustrated), the power supply unit 140, the high-frequency generation means 160, the driving unit 165 and the temperature sensor (not illustrated) to control the operation thereof.

The power supply unit 140 may be electrically connected to the high-frequency generation means 160, and although not illustrated in the drawings, it may be directly connected to the control unit 151 so as to supply a predetermined power to the high-frequency generation means 160 by the control unit 151.

Although not illustrated in the drawings, the input unit 191 and the display unit 192 may be installed in the treatment device body 110. The display unit 192 may display the magnitude of voltage and electric current, and a frequency of high-frequency energy output to a plurality of the needles 121, the temperature of the endothelium adsorption means 170, and the like.

The input unit 191 is provided with a switch (not illustrated), etc. for inputting operation commands. According to user's input, the control unit 151 may control the temperature control means (not illustrated), the air flow means (not illustrated), the high-frequency generation means 160, the driving unit 165, the insertion depth of the needle into the internal tissue surface or the endothelium surface, so as to operate the endosurgical operating device 100.

Although not illustrated in the drawings, the temperature control means (not illustrated) may be installed on the other end of the air supply tube 132, and a suction port (not illustrated) may be formed in the other end portion of the air supply tube 132. The temperature control means is a means for controlling the temperature of the air outside of the operation tube 111 that flows through the suction port. Since the means for regulating air temperature is well known to those skilled in the art, it will not be described.

An air flow means (not illustrated) is installed on the other end of the air exhaust tube 133, and an exhaust port (not illustrated) may be formed in the other end portion of the air exhaust tube 133. The air flow means is a means for making the air in the air exhaust tube 133 flow by force in the exhaust port direction, and may use an air pump, or the like.

When operating the air flow means having the above-described connection relation, air outside of the housing 110 flows in through the suction port 136 to have temperature controlled by the temperature control means, and then it may be made to flow so as to be discharged out of the treatment device body 110 through the exhaust port via the air supply tube 132, the air flow space 172 of FIG. 7, and the air exhaust tube 133.

In this process, the air in the air flow space 172 of FIG. 7 is discharged out of the treatment device body 110 by force, and air is introduced through the suction port as much as the discharged amount, such that the pressure in the air flow space 172 of FIG. 7 may be formed lower than the pressure of the outside. In particular, a difference between the air flow space 172 of FIG. 7 and the outside may be increased as the flow speed of air becomes faster due to the air flow means (not illustrated).

Meanwhile, although not specifically illustrated in the drawings, a ventilating hole (not illustrated) may be formed in the treatment device body 110 so as to communicate the space formed therein and the outside with each other. The ventilating hole may discharge heat generated from the high-frequency generation means 160 and the control unit 151 so as to prevent the heat from being accumulated in the treatment device body 110, which cause an increase in temperature. The number of ventilating holes may be increased or decreased as necessary.

The temperature sensor (not illustrated) may be installed so as to be connected to the endothelium adsorption means 170 for detecting the temperature of the endothelium adsorption means 170. That is, the temperature sensor (not illustrated) may be disposed so as to abut the endothelium adsorption means 170 or may be installed so as to be thermally connected to the endothelium adsorption means 170 through a heat transmission medium (not illustrated).

For reference, the control unit 151, the high-frequency generation means 160, the air flow means and the temperature control means may be disposed in a suitable place among the treatment device body 110 and the control and supply unit 150 of FIG. 2. The displacement place may be selected in consideration of the size of the components or the size or weight of the treatment device body 110.

FIG. 4 is a cross-sectional view for describing the operation of the endosurgical operating device according to the first embodiment of the present invention.

When the plurality of needles 121 protruding from the operation tube front end part 112 penetrate the epithelial layer 11 and reach the lamina propria 13, and then high-frequency electric energy generated from the high-frequency generation means 160 is transmitted to the lamina propria 13 through the plurality of needles 121, the collagen ingredient included in the lamina propria 13 may be coagulated.

At this time, the coagulated portion of the lamina propria 13 may have a bulb shape about the pointed tip 129. As described above, this is the same as the Na-effect and Na-plus effect.

That is, the Na-effect which is also defined in the present disclosure principally refers to forming a double coagulation condition in the lamina propria 13. As wordings for describing the "Na-effect", there may be expressions such as "Burn the tissue of the lamina propria 13, ""Apply heat to the tissue of the lamina propria 13,"and "Boil the tissue of the lamina propria 13." Of these, the expression "Boil the tissue of the lamina propria 13"may be considered to show relatively well the technical meaning of the "Na-effect" which principally refers to 'Apply energy to make the tissue of the lamina propria 13 into a coagulation state.'

Thus, in the present disclosure, the expression, "Boil the tissue of the lamina propria 13" will be principally used to mean 'Apply energy to make the tissue of lamina propria 13 into a coagulation state.'

Meanwhile, "Boil" described in the present disclosure is used to mean "Applying energy" in some cases. However, the inventor of the present invention makes it clear that "Boil" was principally used to mean 'Apply energy to make the lamina propria tissue into a coagulation state.

The Na-effect refers to the effect whereby the epithelial layer 11 is hardly boiled as illustrated in FIG. 4, but a boiled portion 19 is formed only in the portion near the lamina propria 13 in a bulb shape about the tip of the needle 121.

In addition, according to the Na-effect, despite using bipolar electrodes, the whole zone between needle 121 and needle 121 is not boiled but independently boiled space is formed between the respective needles 121.

In other words, according to the Na-effect, the zones between the needles 121 are not continuously boiled as illustrated in FIG. 2, but are boiled differently from each other about the tip of each needle 121. Therefore, it is possible to operate by dividing the tissue minutely using the method of controlling the current intensity and the intervals between the needles 121.

Meanwhile, according to the Na-effect, the operator can expand the bulb-shaped boiled portion by increasing the current intensity applied to the needle 121, and thereby a dumbbell-shaped boiled portion may be formed as two neighboring boiled portions meet each other.

In order to achieve the Na-effect, it is indispensably required that the current should be alternating current forming bipolar electrodes and transmit energy in high-frequency form.

In addition, the intervals between the needles 121 are important for the Na-effect, because if the intervals between the needles 121 are too narrow, the boiled portions concentrated on the tips of the needle 121 stick together to make it difficult to achieve the Na-effect.

Meanwhile, it is preferable in achieving the Na-effect that a net shape is formed when connecting the ends of the needles 121 horizontally and laterally arranged with each other in longitudinal and lateral directions.

Theoretically, a net shape is formed by four needles 121 forming rectangular vertices, but it is preferable that four or more needles 121 are horizontally and laterally arranged, respectively.

In addition, it is preferable in achieving the Na-effect that alternating current is applied to the needles 121, and they are disposed as illustrated in FIG. 7 such that polarity of + and to between the neighboring needles 121 becomes different from each other.

Meanwhile, the alternating current, which is an essential element in achieving the Na-effect, is current of which the intensity and direction change periodically with time and it is usually represented as AC.

For the waveform of such AC, a sine waveform is the most typical, and modification to a square waveform or triangular waveform is possible. Meanwhile, AC is generally used in each country with the frequency standardized to 50 Hz or 60 Hz.

AC is recognized as the most important configuration in producing the Na-effect. Frequency being high does not make any problem unless it is as high as hundreds of Hz, but even if frequency is high, the effect due to it may be offset by narrowing the intervals between the needles 121.

However, low frequency may be considered as a problem in achieving the Na-effect, but it is possible to achieve the Na-effect by controlling the other factors if the frequency is at least 20 Hz or more. Controlling the other factors will be described below.

As a result of multilateral checking and testing, it can be seen that the Na-effect was not achieved without high frequency. For instance, the Na-effect was not reliably achieved if other methods such as ultrasonic waves, an intermediate frequency and ions were used, in addition to a low frequency.

According to experiments, the Na-effect appeared well especially on high frequency of 0.5 MHz or more. It can be seen about 2 MHz to be most preferable, which is currently used.

In short, the range of high frequency for the Na-effect is 0.5 to 10 MHz, preferably 1 to 4 MHz, and more preferably, 1.5 to 2.5 MHz.

To explain this theoretically in more detail, the high frequency lets the energy field form only in relatively close vicinity when the same current was sent to flow, whereas the low frequency lets the energy field form to the part far from the needle 121.

For instance, in the case of the frequency being 0.5 MHz or less, the energy field is too wide even if the interval between the needles 121 are increased, thus it may cause a burn to the epithelial layer 11, and especially in the case that precise treatment is necessary in a narrow tissue portion, it may affect adversely.

Meanwhile, in the case of the frequency being 10 MHz or more, the energy field is too narrow, thus the treatment time becomes long and also it becomes difficult to make an energy field for optimum treatment.

Next, the Na-effect is closely related to the interval of needles 121. Especially the interval between the needles 121 has deep relationship with all other components. For instance, when examining the relation with the frequency of high-frequency current, it can be seen through experiments that the interval between the needles 121 should be decreased if the frequency of high-frequency current increases and the interval between the needles 121 should be increased if the frequency of high-frequency current decreases, in order to maintain the Na-effect.

Meanwhile, in the case of the frequency being 2 MHz, it can be seen that the most suitable interval between the needles 121 is about 2 mm. This will be described below in more detail.

Next, as a component that affects the Na-effect, it can take whether the needles 121 is coated for insulation. In principle, as long as the Na-effect is in operation, the proximity of the epithelial layer 11 is hardly boiled, and a boiled portion similar to an electric bulb is formed about the tip of the needle 121 that has dug into the lamina propria 13, as illustrated in FIG. 4. Therefore, it is not necessary to do extra insulation coating.

However, as long as insulation is not taken as a purpose, it cannot be deemed that the Na-effect is hampered by coating itself. For example, it was confirmed that metal coating or coating by heat treatment which may not affect insulation while reinforcing the strength of the needle 121 does not affect the Na-effect.

In other words, it is preferable that insulation coating is not performed in order to maximally utilize the Na-effect, and it is preferable that insulation coating is not performed at least on the section of the lamina propria 13 even if the insulation coating is performed.

Another component that has close relationship with the Na-effect is a method of disposing the polarity of the needle 121 (that is, electrode). In principle, alternating current is applied to achieve the Na-effect, but it is preferable to dispose such that the polarities between the neighboring needles 121 are different from each other.

This means that the plurality of needles 121 are disposed equally in the longitudinal and lateral directions, and in the case that each needle 121 is located at the intersection of the square network structure, the polarity of the needle 121 at any position is different from the polarity of the needle 121 at the nearest intersection.

Of course, since alternating current is used, the polarity of each needle 121 is changed 50 to 60 times per second when using ordinary alternating current. Even so, alternating current should be applied to the needle 121 obviously every minute and it is preferable to dispose such that the polarities between the neighboring needles 121 are different from each other.

That is, in the present invention, arranging the polarity of each needle 121 while alternating to (+) and (-) in each direction so as to be different from the polarity of the most closely neighboring needle 121 means that it is configured in such a principle.

Of course, it is preferable that each needle 121 is disposed so as to be located at the intersection in a square network structure. However, even if the needles 121 are disposed so as to be located at intersections in a rhombus-shaped network structure, rectangular network structure or other quadrangular network structure, it can be seen that the Na-effect is generated as long as the needles 121 are disposed such that the polarity between the closest neighboring needles 121 are different from each other.

Meanwhile, in embodying the present invention, the needles 121 without electricity may be partially disposed among the needles 121, and the needles 121 without electricity may not be partially disposed. However, even in this case, if the polarity of the needles 121 is disposed such that the polarities of the most closely neighboring needles 121 are different from each other, the Na-effect may be obtained as a matter of course.

In operation of the Na-effect, another important component is voltage. Voltage is an important element to be considered in determining the interval between the needles 121, but it is also a component directly related to the safety of equipment. Therefore, it is preferable to measure with the focus on the voltage applied to the tissue surface.

"Voltage actually applied to the tissue" is the voltage applied between tissue portions in contact with the surface of the needle 121 inserted into the endothelial tissue. Thus, unlike the voltage applied to equipment, it may vary with the sum of three resistance values (i.e., equipment resistance, electrode (needle) resistance, and tissue resistance values).

This is a component directly related to the quantity of energy applied, and it is preferable to not exceeding maximum 100 V. Meanwhile, preferably, voltage is 10 to 60V, and optimum voltage was confirmed to be 20 to 40V through experiments.

Voltage (tissue voltage) applied to the tissue of the human body during operation may be designed to vary depending on the voltage (external voltage) set in the equipment and the circuit design. This may be easily achieved by those skilled in the art and is not a characteristic component, therefore it will not be specifically described.

Another variable in the Na-effect is electric current. However, current varies with voltage and resistance values, thus if the values of voltage applied through equipment, the instrument resistance, the electrode (needle) resistance, and the tissue resistance are determined, it is possible to calculate the current according to the Ohm's law. Meanwhile, as described above, the resistance values may be classified into the electrode (needle) resistance and tissue resistance.

Yet another important component in the Na-effect is energy duration time. When examining the minimum time, it takes about 0.02 seconds for high-frequency energy to reach a safety zone. Therefore, if energy duration time is too short, the effect is as weak as hardly measurable, so it is not possible to make time exceedingly short.

The results of measuring through experiments showed that the Na-effect is expressed when the time is at least 0.05 seconds or more. A major characteristic of the present invention is that energy duration time is relatively short. However, the longest time at which the effect shows was measured to be 0.8 seconds.

Of course, increasing the interval between the needles 121 and minimizing the voltage can make the duration longer. However, it is preferable to set the time in the range of 0.05 to 0.08 seconds, based on the optimum condition available for operation as of the present point of time.

However, it can be seen by measuring that preferable time is 0.1 to 0.4 seconds, and more preferably, 0.1 to 0.2 seconds. Finding out such times was very difficult, more difficult than the inventor expected. The inventor repeated innumerous trials and errors and needed a lot of insight. It was not until the inventor created the Na-effect that he achieved the applying time should be made short as described above, although the inventor have used high frequency, alternating current, and low voltage.

In the Na-effect, other important components to be considered subsequently are a length and diameter of the needle 121. The diameter of the needle 121 affects the interval between the needles 121. Further, the needle should be inserted into and drawn out of the tissue repeatedly. Therefore, the needle should be able to endure sufficiently without being bent in the process. Meanwhile, as the diameter of the needle 121 increases, pain to a patient may be increased, a big scar made as a result of inserting the needle 121 may be remained, and bleeding may be increased.

Thus, it is preferable that the needle 121 have a diameter so as to minimize pain to the patient during inserting it into the tissue without being bent. Therefore, finding an optimum range to minimize the appearance of scars is required. The diameter of the needle 121 currently in clinical use is 0.25 mm and 0.3 mm, which are considered acceptable for use.

The Na-effect may be expressed even if the needle 121 has a diameter out of the above range slightly. That is, although the diameter of the needle 121 affects the La-effect more or less, the effects thereof are very small.

Meanwhile, one of the things that should pay the most attention in designing equipment that has the Na-effect is the interval between the needles 121 as described above. There are various methods for measuring the interval. However, herein, it will be described with the focus on the shortest interval between the integument of a needle 121 and the integument of a neighboring needle 121. In the present invention, the interval between the needles 121 was defined by the above-described method.

It can be seen by experiments that the shortest interval between the needles capable of obtaining the Na-effect is 1.3 mm. Thus, it can be seen that preferable intervals are 1.3 to 3.0 mm. However, intervals of different ranges with wider intervals are considered to be usable by utilizing the method of reducing the quantity or resistance of the current applied. At this time, the interval between the needles 121 may vary little by little with many variables.

When examining components that affect the interval between the needles 121 under the premise of obtaining proper Na-effects, there are factors as follow. First, if power (=energy) increases or the diameter of needle 121 increases, the interval between the needles 121 should be increased. Meanwhile, since power is the value made by multiplying voltage, current and applied time, the interval between the needles 121 should be increased if at least one of the values of voltage, current, applied time, the diameter of needle 121, and conductivity increases.

However, if at least one of the values of resistance, AC frequency, high frequency and the depth of the needle 121 that has penetrated into the tissue (skin), the interval between the needles 121 may be narrowed further according to the extent of the impact. Even in that case, there is no large impact in obtaining the Na-effect. This may be expressed by Equation 1 below.

[Equation 1]

$$Interval = \frac{(Power(= Energy)) * (Needle\ thickness) * (Conductivity)}{(Resistance) * (AC\ frequency) * (High\ frequency) * (Needle\ depth\ penerated\ into\ sikn)}$$

Wherein, N denotes a proportional constant, and

The resistance denotes equipment resistance, electrode resistance and tissue resistance.

Since power (=energy) J = W*t,

(W=power, t=time)

=V*I*t (W=V*I)

= I$^2$*R*t (V=I*R)

Therefore, the above Equation 1 may also be expressed by Equation 2 below.

[Equation 2]

$$Interval = \frac{V * I * t * Needle\ thickness * Conductivity}{(Resistance) * (AC\ frequency) * (High\ frequency) * (Needle\ depth\ penerated\ into\ sikn)}$$

Meanwhile, as illustrated in the drawing, high-frequency energy may affect not only the lamina propria 13 but also the needle 121, and the epithelial layer 11 in the neighboring zone. At this time, since the impact on the epithelial layer 11 is not intended, minimizing is preferable. This will be described below with reference to FIGS. 7 to 9.

For reference, as described above, the lamina propria 13 is a component of mucosa. Thereby, the lamina propria may not be included in the endothelium. However, since the collagen ingredient is included in most of the tissue below the epithelial layer 11 in the endothelium, description below will be made assuming that the lamina propria 13 is disposed below the epithelial layer 11.

FIG. 6 is a bottom view illustrating the endosurgical operating device according to the first embodiment of the present invention.

Referring to FIG. 6, the plurality of needles 121 may be dispersedly disposed such that the pointed tips 129 are protruded outward of the operation tube front end part 112, on the bottom of the operation tube front end part 112 of the endosurgical operating device 100 of FIG. 1 according to the first embodiment of the present invention, that is, in the other end portion of the operation tube 111, as illustrated.

At this time, the interval between the needles 121 may be equal as illustrated in the drawings, or although not illustrated in the drawings, if necessary, they may be density disposed in a central portion of the adsorption plate portion 173 or a periphery portion or a specific portion.

Of the plurality of penetration holes 174 formed in the endothelium adsorption means 170, the penetration holes formed in the adsorption plate portion 173 may have a larger diameter than the diameter of the plurality of needles 121, as illustrated in the drawings, and may be formed at a suitable interval between the needles 121.

Such an interval allows the pointed tips 129 of the needles 121 easily penetrate the epithelial layer 11 and reach the lamina propria 13. This will be described with reference to FIGS. 7 to 9.

FIGS. 7 to 9 are enlarged views for describing the operation of the endothelium adsorption means.

Referring to FIG. 7, when the endothelium 10 is pressed by the plurality of needles 121 for treatment using the plurality of needles 121, the needles 121 may only press in the epithelial layer 11, failing to penetrate it, since the endothelium 10 is a soft tissue as illustrated.

In this case, when high-frequency electric energy is applied thereto through the plurality of needles 121, only the epithelial layer 11 is affected. Therefore, it is not possible to obtain the treatment effect on the lamina propria 13, but only a side effect of the epithelial layer 11 being denaturalized may be brought about.

FIGS. 7 to 9 are enlarged views of portion B illustrated in FIG. 3 to describe the operation of the endosurgical operating device.

Referring to FIG. 7, the plurality of needles 121 penetrated only the epithelial layer 11 of the endothelium 10, which is a soft tissue, the pointed tip 129 failing to reach the lamina propria (see 12 of FIG. 3). In this case, when high-frequency energy is applied thereto through the plurality of needles 121, the expected improvement effect of the lamina propria 12 of FIG. 3 is very small and a side effect may occur on the epithelial layer 11.

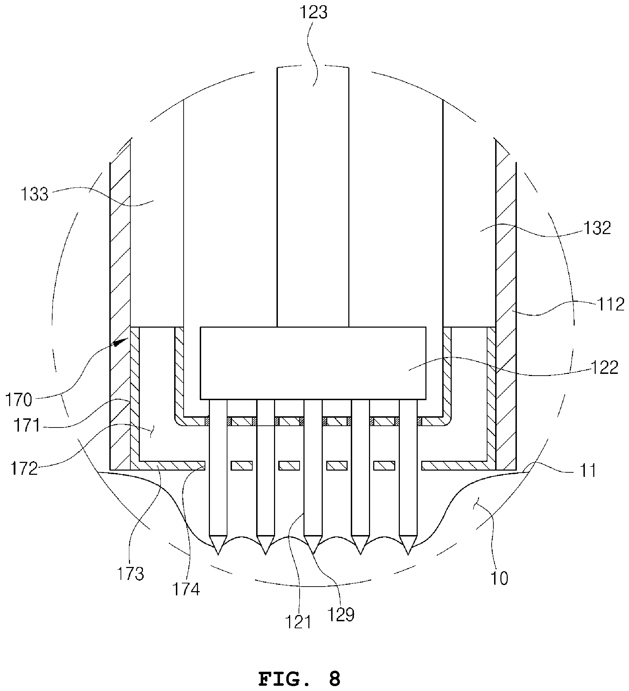

FIG. 8 is an enlarged view illustrating the operation of the endothelium adsorption means 170 when the endothelium 10 is a soft tissue.

Referring to FIG. 8, as air flows in the air flow space 172, the air outside of the housing 110 of FIG. 2, that is, the air outside of the adsorption plate portion 173, is introduced into the air flow space 172 through the intervals formed between the plurality of pins 121 and penetration holes 174.

This is because, as described with reference to FIG. 2, when flow of air is generated in the air flow space 172 by operation of an air flow means (not illustrated), the pressure in the air flow space 172 is decreased.

Therefore, the epithelial layer 11 of the endothelium 10 is adhered to the adsorption plate portion 173 by the flow of air introduced into the penetration holes 174. Accordingly, the plurality of needles 121 sufficiently penetrate the endothelium 10, so that the pointed tip 129 can reach the lamina propria 13. Therefore, it is possible to sufficiently get the treatment effect by high-energy electric energy transmitted through the plurality of needles 121.

Meanwhile, when the air introduced into the air flow space 172 is heated or cooled by the temperature control means (not illustrated), it is possible to control the temperature of the adsorption plate portion 173. When high-frequency energy reaches the lamina propria (see 12 of FIG. 3), temperature rises. At this time, if the temperature of the adsorption plate portion 173 is decreased, it may be prevented that the temperature of the epithelial layer 11 rises together. Therefore, denaturalization of the epithelial layer 11 by high-frequency energy may be prevented.

Further, although not illustrated in the drawings, blood capillaries distributed in the lamina propria 13 are destroyed to cause bleeding, during the lamina propria 13 being penetrated by the plurality of needles 121. In such a case, when decreasing the temperature of the adsorption plate portion 173, the upper layer of the lamina propria 13 is also decreased. Therefore, circulation through destroyed capillaries is decreased, an effect of hemostasis or decreasing blood loss may be obtained.

The control unit 151 may continuously measure the temperature of the endothelium adsorption means 170 detected by the temperature sensor (not illustrated), so as to maintain the temperature of the endothelium adsorption means 170 within a predetermined range. For reference, the denaturalization prevention effect and the bleeding suppression effect of the epithelial layer 11 may be obtained with a range of 10 to 25 degrees Celsius. By previously inputting the temperature range into the control unit 151 and controlling the operation of the air flow means (not illustrated) and the temperature control means (not illustrated), it is possible to maintain the temperature of the adsorption plate portion 173 of the endothelium adsorption means 170 within the above-described range.

For reference, the plurality of needles 121 once used in operation for treatment need to be replaced with new needles for reasons of sanitation. As described above, if the operation tube front end part 112 is configured separately from the other end of the operation tube 111, it is possible to easily replace the needle module 120 only.

Meanwhile, although not illustrated in the drawings, in the endosurgical operating device 100 as described above, the other end portion of the air supply tube 132 and the other end portion of the air exhaust tube 133 are connected to each other so as to be able form a closed circuit. A filtration means (not illustrated), an air flow means (not illustrated) and a temperature control means (not illustrated) may be installed on the closed circuit.

Therefore, when operating the air flow means, air in the air exhaust tube 133 moves in the above-described closed circuit. At this time, air may flow into the air flow space 172 formed in the endothelium adsorption means 170 via the temperature control means and goes through the endothelium adsorption means 170, and then it may be introduced into the filtration means through the air exhaust tube 133.

At this time, the filtration means filters blood or body fluid such as gastric juice when it flows into the endothelium adsorption means 170, so that it plays a role of reducing the contamination possibility and contamination range of the components besides the air exhaust tube 133.

As the filtration means, a variety of filters may be used such as a porous filter for adsorbing blood or body fluid mixed into air, a sterile filtration filter for preventing germs from passing and wet filtering equipment. Herein, the wet filtering equipment includes a tank filled with purified water or antiseptic solution. The tank may be configured in such a manner that the bottom thereof is connected to the other end of the air exhaust tube 133, and air leaking from the air exhaust tube 133 passes through purified water or antiseptic solution, and then air is collected by the method such as water substitution to be introduced into the air flow means.

During air passing through purified water or antiseptic solution, it is possible to filter blood or body fluid included in air or fine foreign substances introduced through the penetration hole 174 of the endothelium adsorption means 170. Air passing through the filtration means goes through the circulation process as described above again through the air flow means.

Thus, as the air supply tube 132 and the air exhaust tube 133 form the closed circuit, blood or tissues that dropped out of the skin of the patient do not leak out of the closed circuit even if they were introduced into the air exhaust tube 133. Therefore, the possibility of causing infection may be greatly reduced. In addition, by adding the filtration means as described above, sanitation may be further improved.

For reference, the filtration means, the air flow means and the temperature control means which are installed in the closed circuit formed by the air supply tube 132 and the air exhaust tube 133 may have the disposition sequence changed so as to change the passing sequence of flowing air. However, if the air passing through the endothelium adsorption means 170 is allowed to pass through the filtration means first, it is possible to greatly decrease the possibility that the air flow means and temperature control means are contaminated by blood or body fluid.

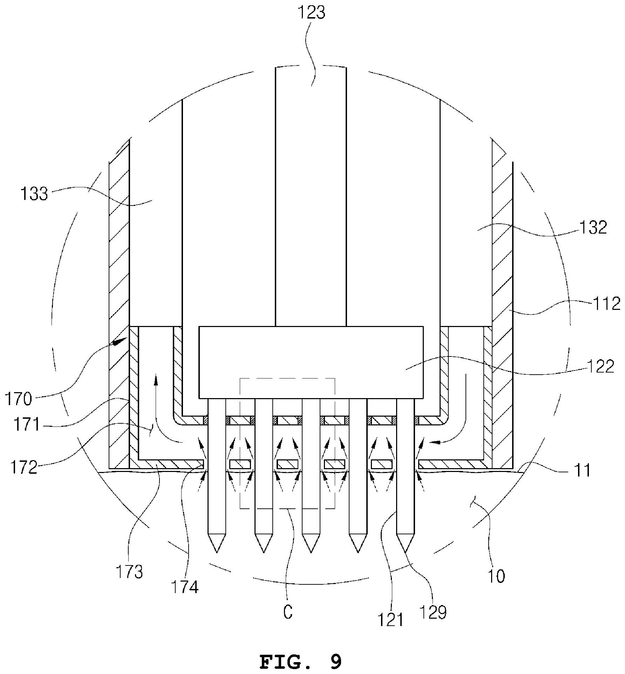

FIG. 10 is an enlarged view of portion C illustrated in FIG. 9.

Referring to FIG. 10, in the penetration holes 174 and 175 formed in the adsorption means body 171, an insulation member 179 may be coupled to an edge portion of the penetration hole 175, excluding the penetration holes disposed in the adsorption plate portion 173, that is, the penetration hole 175 that is not exposed to one side of the housing 110 of FIG. 2.

The insulation member plays a role of preventing the high-frequency electric energy that is applied thereto through the plurality of needles 121 from being transmitted to the adsorption means body 171. That is, the adsorption means body 171 may be made of a thermal conductor. A heat conductor may be an electric conductor as well, thus if high-frequency energy is applied to the adsorption means body 171, the high-frequency electric energy may be transmitted to the epithelial layer 11 therethrough.

Therefore, when the plurality of needles 121 and the adsorption means body 171 are electrically and thermally insulated from each other by using the insulation member 179, it is possible to prevent an occurrence of the side effect as described above.

If a difference of the diameter between the plurality of penetration holes 174 and the needle 121 is small, the outer circumference surface of the needle 121 may come into contact with the inner circumference surface of the penetration hole 174 during the needle 121 passing through the skin. To prevent this, although not illustrated in the drawings, the insulation member 179 may also be installed in the penetration hole 174. Alternately, although not illustrated in the drawings, when the possibility that the outer circumference surface of the needle 121 will come into contact with the inner circumference surface of the penetration holes 174 and 175 is low like in the case that a difference of the diameter between the needles 121 of the penetration holes 174 and 175 is high, the insulation member 179 may not be coupled with the penetration holes 174 and 175.

Meanwhile, although not illustrated in the drawings, one or more of the needles 121 may have a hollow portion formed like a syringe needle. In the needle module 120, the portion without the pointed tip 129 of the needle 121 formed, the operation plate 122 or the connection member 123 may also be provided with a drug container (not illustrated) connected to the hollow portion (not illustrated). The drug container A may be provided with a small actuator controlled by the control unit 150 therein, and the actuator may be provided with a piston which presses drug toward the hollow portion at an end portion thereof.

Therefore, when the needle 121 with the drug container (not illustrated) having a specific effect injected penetrates the endothelium 10, the drug is transmitted into the endothelium 10 through the hollow portion (not illustrated), so that it is possible to get the effect by the drug other than the treatment effect by the needle 121.

For instance, when a scar or ulcer occurred in the endothelium 10, if a growth factor is used as medicine to treat the ulcer, it is possible to obtain an effect of facilitating the recovery of the scar or ulcer. That is, when a scar or ulcer occurred in the endothelium 10, autotherapy proceeds to the central portion from the edge of the scar or ulcer.

However, if energy is supplied to the scar or ulcer by using the endosurgical operating device 100 according to the present embodiment, cure may progress quickly as cure proceeds in the energy-supplied portion as well as the edge portion. In addition, as described above, injecting a growth factor into the portions of scar or ulcer may make cure proceed more quickly.

Meanwhile, the needle 121 may be made of various materials. If the needle 121 is made of metal or ceramic, costs thereof may be increased, but cooling effect in the upper portions of the epithelial layer 11 and the lamina propria 13 may be increased. If the needle 121 is made of synthetic resin, heat conductivity becomes relatively lower than the needle made of metal or ceramic, so that the cooling effect may be decreased, but costs thereof may be largely decreased. Thus, the needle 121 may be made of any material that is selected according to use.

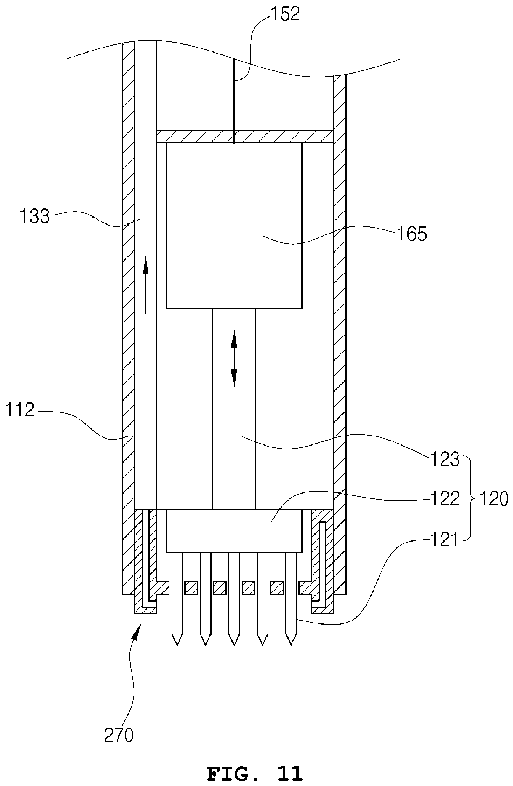

FIG. 11 is a longitudinal sectional view illustrating the tip portion of an endosurgical operating device according to a second embodiment.

The operation tube front end part 112, the needle module 120, the power transmission member 152, the driving unit 165, the air flow means (not illustrated) and the temperature sensor (not illustrated) included in the endosurgical operating device according to the second embodiment are the same as described above, thereby duplicate parts will not be described.

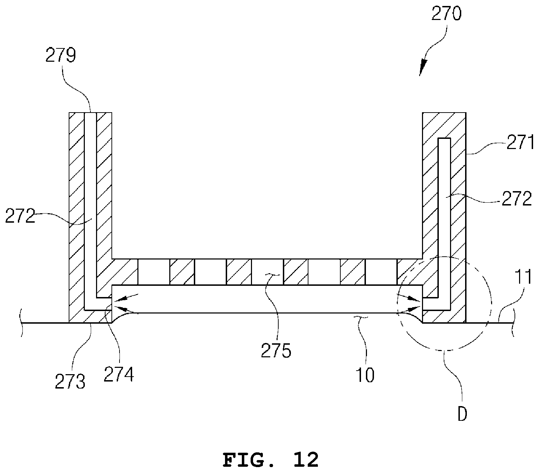

The endosurgical operating device according to the second embodiment may have only the air exhaust tube 133 without having the air supply tube (See 132 of FIG. 3) as described above. That is, air moved by the air flow means (not illustrated) installed in the air exhaust tube 133 may be made to flow in through a skin adsorption means 270, which will be described with reference to FIG. 12.

FIG. 12 is a longitudinal sectional view illustrating the endothelium adsorption means illustrated in FIG. 11. The endothelium adsorption means will be described with reference to FIGS. 11 and 12.

The epithelium adsorption means 270 includes an adsorption means body 271. The adsorption means body 271 has a shape that surrounds a part of the operation plate 122. The adsorption means body 271 has a plurality of penetration holes 275 through which the plurality of needles 121 penetrate formed therein. An air flow space 272 is formed in the adsorption means body 271.

In addition, the adsorption means body 271 has an adsorption part 273 formed therein so as to protrude in a direction abutting the endothelium 10. An inlet port 274 is formed in the adsorption part 273. A connection portion 279 connected to the air exhaust tube 133 is formed on one side of the adsorption means body 271.

The air flow space 272 may be formed inside of the edge portion of the adsorption means body 271 as illustrated. The air flow space 272 is formed in a shape connecting the connection portion 279 of the adsorption means body 271 and the inlet port 274, so that air may flow from the connection portion 279 into the inlet port 274

Herein, although not specifically illustrated in the drawings, the portion of the adsorption part 273 abutting the endothelium 10 may be formed so as to have a polygon or closed curve shape. That is, when the adsorption part 273 comes into contact with the endothelium 10, and if the adsorption part 273 and the portion in which the adsorption part 273 and the endothelium 10 come into contact are connected imaginarily, a shape of figure, in which the inside and the outside are isolated from each other by a line segment or curve that are connected to each other like a polygon or closed curve, may be obtained.

For instance, if the plurality of needles 121 are disposed as illustrated in FIG. 6, the portion in which the adsorption part 273 comes into contact with the skin may have a rectangular shape. The portion in which the adsorption part 273 and the endothelium 10 come into contact with each other may have not only a polygonal shape such as a triangle or rectangle, but also a closed curve shape such as the circumference of a circle or ellipse.

Therefore, if the adsorption means body 271 abuts the endothelium 10, the abutted portion has a shape covered with the portion in which the penetration hole 275 of the adsorption means body 271 is formed and the adsorption part 273 of a shape protruded from this.

The inlet port 274 may be formed in a portion facing the inner direction of the above-described polygon or closed curve, in the portion that does not directly come into contact with the endothelium 10 in the adsorption part 273.

Therefore, when air flows through the air exhaust tube 133, the air flows into the air exhaust tube 133 through the connection portion 279 via the air flow space 272 from the inlet port 274. At this time, since the needle 121 as described above is inserted into the penetration hole 275, air flows out mainly through the inlet port 274. Thereby, negative pressure acts on the portion covered with the adsorption means body 271 in the endothelium 10.

Therefore, the epithelial layer 11 may protrude in the direction of the penetration hole 275, and the needle 121 may easily penetrate the epithelial layer 11.

If bleeding occurs during treating the endothelium 10 by penetrating the needle 121 into the epithelial layer 11 as described above or body fluid in the vicinity flows in, blood or body fluid may flow in through the inlet port 274. The blood or body fluid flowing through the inlet port 274 may flow to the air exhaust tube 133 through the air flow space 272.

As described above, when replacing the operation tube front end part 112 after the treatment for the endothelium 10 is completed, the epithelium adsorption means 270 may be replaced as well to solve the above-described problems. At this time, it is possible to prevent the air exhaust tube 133 from being infected by blood by providing it with a filter or filtration means as described above.

For reference, in order to easily replace the air exhaust tube 133, unlike what is illustrated, the air exhaust tube 133 may be disposed outside of the operation tube 111 and the operation tube front end part 112, and then may be detachably coupled to the connection portion 279 in a shape in which one end portion of the air exhaust tube 133 penetrates the operation tube front end part 112. This may also be applied to the air supply tube 132 of FIG. 3.

That is, the air supply tube 132 and the air exhaust tube 133 are used as disposable tubes, and the air supply tube 132 and the air exhaust tube 133 are not disposed in the operation tube 111, but are disposed together with operation tube 111 through the channel 6 of FIG. 2 formed in the insertion part 5 of FIG. 2. Then, since the air supply tube 132 and the air exhaust tube 133 may be discarded after the treatment for the endothelium 10 is completed, sanitation may be further improved.

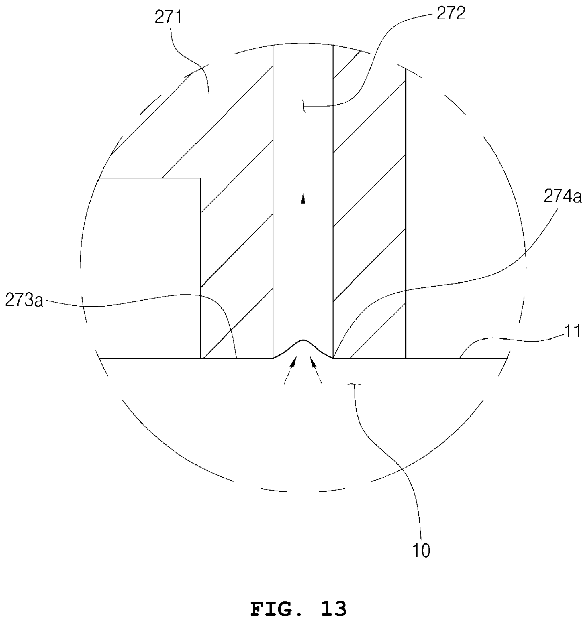

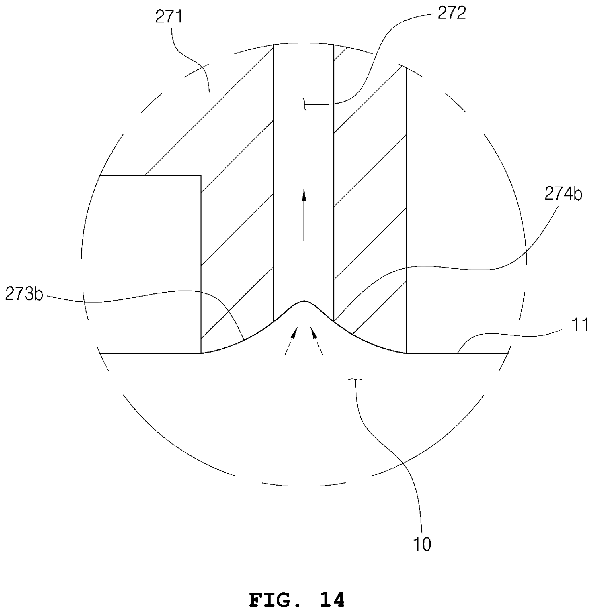

FIGS. 13 and 14 are enlarged views illustrating a modified example of the adsorption part of the epithelium adsorption means.

For reference, FIGS. 13 and 14 are enlarged view of the portion corresponding to portion D of the epithelium adsorption means 270 illustrated in FIG. 12.

Referring to FIG. 13, an adsorption hole 274a is formed in a portion in which an adsorption portion 273a and the endothelium 10 come into contact with each other. That is, as illustrated in FIG. 13, the adsorption hole 274a may be formed toward the direction abutting the epithelial layer 11 from the air flow space 272 of the epithelium adsorption means 270.

Therefore, when air flows in through the adsorption hole 274a as air is discharged through the air exhaust tube 133 as described above, the epithelial layer 11 may be directly adsorbed into the adsorption hole 274a to be fixed on the adsorption portion 273a, as illustrated in FIG. 13. Accordingly, the effect of the epithelium adsorption means 270 fixing the endothelium 10 may be improved.

Referring to FIG. 14, an adsorption hole 274b is formed in a portion in which an adsorption portion 273b abuts the endothelium 10. The abutted portion has a shape recessed concavely toward the adsorption hole 274b. That is, the portion of the adsorption portion 273b abutting the endothelium 10 may have a shape like a funnel as a slope is formed toward the inside of the air flow space 272 as it goes closer to the adsorption hole 274b.

Therefore, when air flows in through the adsorption hole 274b as air is discharged through the air exhaust tube 133, the epithelial layer 11 is adsorbed to the adsorption hole 274b as illustrated in the drawings, and a part of the epithelial layer 11 is fixed to the concavely recessed portion of the adsorption portion 273b. Accordingly, the effect of the epithelium adsorption means 270 fixing the endothelium 10 may be further improved.

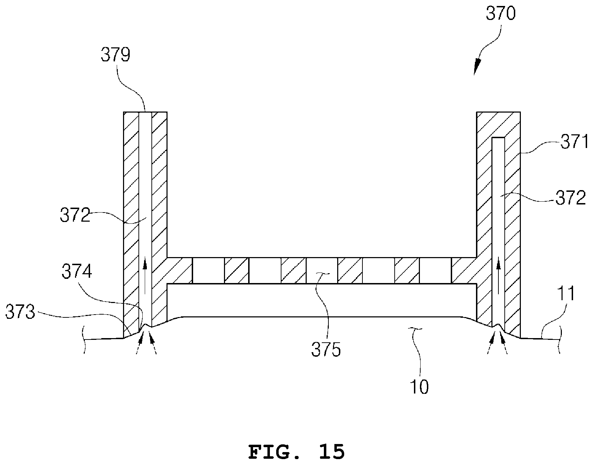

FIG. 15 is a longitudinal sectional view illustrating a modified example of the epithelium adsorption means.

The epithelium adsorption means 370 includes an adsorption means body 371. An air flow space 372, an adsorption part 373, a plurality of penetration holes 375 and a connection portion 379 are formed in the adsorption means body 371. Herein, the air flow space 372, the plurality of penetration holes 375 and the connection portion 379 are the same as the air flow space 272, the plurality of penetration holes 275 and the connection portion 279 described with reference with FIG. 12, thereby duplicate parts will not be described.

The adsorption part 373 of the epithelium adsorption means 370 is also formed so as to protrude from the adsorption means body 371 toward the direction abutting the endothelium 10 likewise with the adsorption part 273 of FIG. 12 described above. In addition, it may be formed such that the portion of the adsorption part 373 that abuts the endothelium 10 has polygon or closed curve shape.

An adsorption hole 374 is formed in the portion in which the adsorption part 373 and the endothelium 10 come into contact with each other. That is, the adsorption hole 374 may be formed toward the direction abutting the epithelial layer 11 from the air flow space 372 of the epithelium adsorption means 370.

When air flows in through the adsorption hole 374 as negative pressure acts in the air flow space 372 as described above, the epithelial layer 11 may be directly adsorbed to the adsorption hole 374 to be fixed on the adsorption part 373.

At this time, a slope may be formed in the portion that abuts the endothelium 10 of the adsorption part 373 as illustrated. This slope has a shape in which the height of the adsorption part 373 protruding toward the endothelium 10 from the adsorption means body 371 decreases, as it faces the direction in which penetration holes 375 are disposed, that is, the direction in which the needle module 120 is disposed.

Therefore, as illustrated in the drawings, the epithelial layer 11 has an elevated shape as it goes to the middle portion of the adsorption means body 371 by the slope formed in the adsorption part 373 during the epithelial layer 11 is fixed to the adsorbed portion 373. Accordingly, the epithelial layer 11 may protrude in the direction of the penetration holes 375 as illustrated in the drawings, and the plurality of needles 121 may easily penetrate the epithelial layer 11.

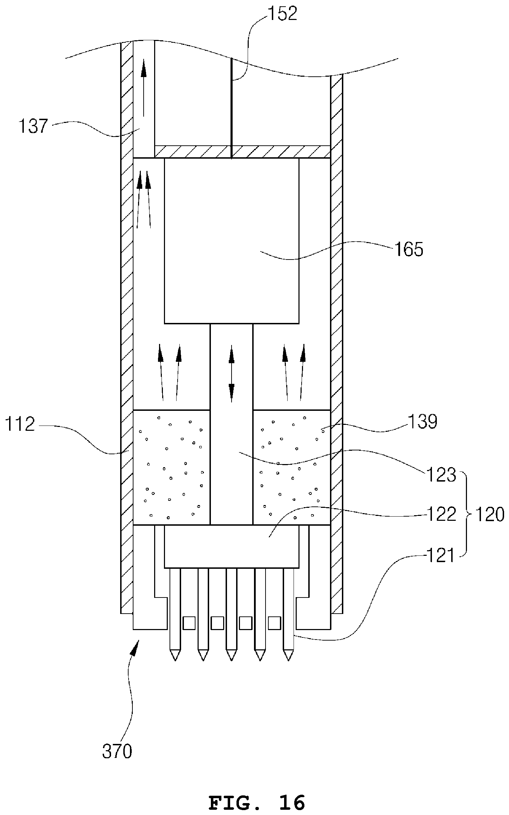

FIG. 16 is a longitudinal sectional view illustrating the tip portion of the endosurgical operating device according the third embodiment. The tip portion will be described with reference to FIG. 15.

The endosurgical operating device according the third embodiment is different only in the configuration of the exhaust tube 137 and the filter 139 compared to the endosurgical operating device according to the second embodiment described with reference to FIG. 12, and the other components are the same. Therefore, duplicate parts will not be described.

The epithelium adsorption means 370 is installed in the operation tube front end part 112. The epithelium adsorption means 370 is installed such that part thereof penetrates the operation tube front end part 112 to be exposed to the outside.

One side of the exhaust tube 137 may be disposed in the operation tube front end part 112 as illustrated. In this case, if an air flow means (not illustrated) is operated for the endothelium 10 to be adsorbed by the epithelium adsorption means 370, negative pressure is generated in the operation tube front end part 112, thereby blood or body fluid as described above may flow into the plurality of needles 121 and between the plurality of penetration holes 375.

In such a case, there is a possibility that the inside of the operation tube front end part 112 is contaminated by blood or body fluid. Therefore, if the filter 139 is disposed in the operation tube front end part 112 in a form surrounding the needle module 120 as illustrated in the drawings, blood or body fluid that flow into the operation tube front end part 112 together with air may be adsorbed to the filter 139.

After treatment for the endothelium 10 is completed, spread of contamination by blood or body fluid may be prevented by separating the operation tube front end part 112 from the operation tube 111 as described above and discarding the needle module 120, the epithelium adsorption means 370 and the filter 139.

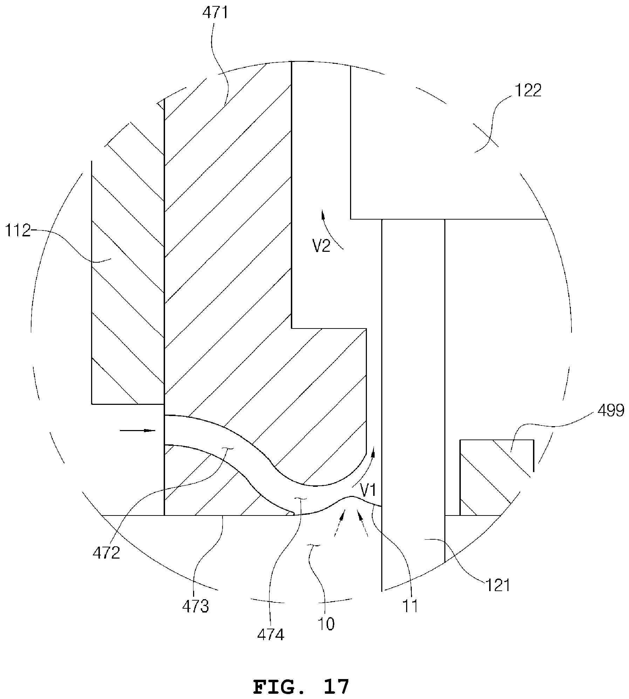

FIG. 17 is an enlarged view illustrating an adsorption part of an endosurgical operating device according to a fourth embodiment.

A portion through which the operation plate 122 and the plurality of needles 121 penetrate is formed in the middle portion of the adsorption means body 471 included in the epithelium adsorption means. That is, the adsorption means body 471 has a shape partially surrounding the edge portion of the operation plate 122.

A surface temperature control means 499 is disposed between the plurality of needles 121 in the middle portion of the adsorption means body 471, that is, the portion through which the plurality of needles 121 penetrate. Although not illustrated in detail, the surface temperature control means 499 is fixed on the adsorption means body 471, and when the adsorption means body 471 comes into contact with the epithelial layer 11 of the endothelium 10, it is also disposed so as to abut the surface temperature control means 499. The surface temperature control means 499 may use thermoelectric semiconductor elements, etc., and may be electrically connected to the control unit 151 or the power supply unit 140. The control unit 151 may to control the temperature of the epithelial layer 11 by controlling power supplied thereto.

An air flow space 472 may be formed in the adsorption means body 471. As illustrated in the drawings, one side of the air flow space 472 is open toward a side of the adsorption part 473 and the other side is formed so as to be connected to the adsorption hole 474 formed in the portion abutting the endothelium 10 of the adsorption part 473. Air passing through the air flow space 472 to go through the adsorption hole 474 may flow to the exhaust tube 137, after passing through the space formed between the adsorption means body 271 and the needle module 120 to go through the internal space of the housing 110.

Herein, the side of the adsorption part 473 with one side of the air flow space 472 open refers to a direction opposite to the direction in which the needle module 120 is disposed in the side of the adsorption part 473. Therefore, when the adsorption means body 471 comes into contact with the endothelium 10, the air outside of the space formed by the adsorption part 473, the needle 121 and the surface temperature control means 499 may flow into one side of the air flow space 472.

According to the above-described configuration, when the air in the housing 110 starts to be discharged out through the exhaust tube 137 by operating the air flow means (not illustrated), negative pressure acts on the inside of the housing 110. Therefore, air flows in the air flow space 472 as shown by the arrows in the drawing.

In the present embodiment, as illustrated in the drawings, the air flow channel formed between the adsorption means body 471 and the needle module 120 has a larger cross-sectional area than the cross-sectional area of the air flow space 472. Therefore, the velocity V1 at which air flows in the air flow space 472 is higher than the velocity V2 at which air flows between the adsorption means body 471 and operation plate 122.

Bernoulli effect is generated by such a difference of air flow velocity, so that the air pressure of the other side portion of the air flow space 472 becomes lower than the air pressure between the adsorption means body 471 and the operation plate 122. Accordingly, as illustrated in the drawings, the epithelial layer 11 of the endothelium 10 abutting the adsorption part 473 is adsorbed into the other side portion of the air flow space 472. Therefore, the endothelium 10 may be fixed by the epithelium adsorption means body 470.

As described above, bleeding may occur during treating the endothelium 10, and also in the present embodiment, blood may flow in according to the flow of air through the space between the adsorption means body 471 and the operation plate 122. In order to prevent the inside of the housing from being contaminated by the blood flowing in, a filter 139 as described above may be provided in the operation tube front end part 112.

For reference, in the case of a very weak or thin tissue, too strong force for adsorbing the endothelium 10 may cause damage to the tissue. Therefore, although not illustrated in the drawings, the embodiments of the present invention may be configured in such a manner that a pressure sensor for directly or indirectly measuring the negative pressure applied to the skin by air flow means is added, and the action intensity of the air flow (not illustrated) is controlled by the control unit 151 depending on the pressure measured by the pressure sensor.

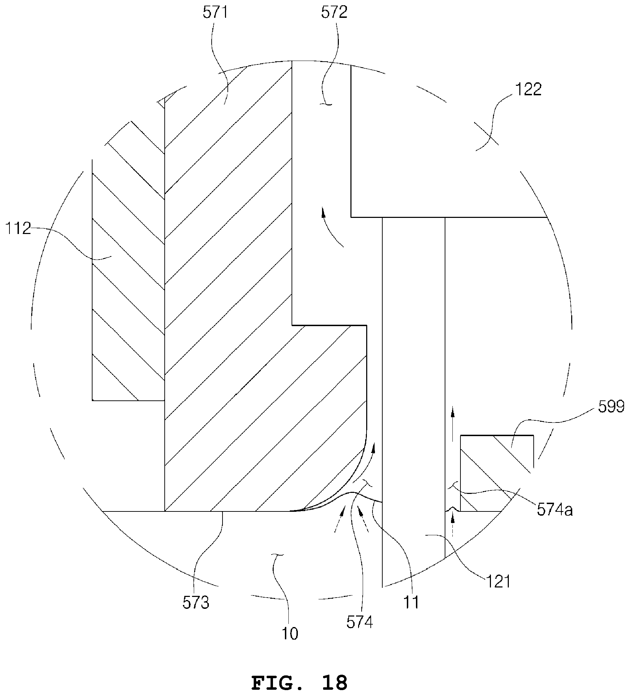

FIG. 18 is an enlarged view illustrating an adsorption part of an endosurgical operating device according to a fifth embodiment.

The adsorption means body 571 included in the endosurgical operating device according to the fifth embodiment is configured in such a manner that one side of the air flow space 472 is closed compared to the adsorption means body 471 described with reference to FIG. 17. Since the configuration other than that is the same, duplicate parts will not be described.

Therefore, the air flow space 572 of the adsorption means body 571 may be formed between the adsorption means body 571 and the operation plate 122 as illustrated in FIG. 18, and the adsorption holes 574 and 574a may be formed between the adsorption part 573 formed in the adsorption means body 571 and the needle 121, and between the needle 121 and a surface temperature control means 599

Therefore, when the air flow means (not illustrated) is operated, it is possible to make the epithelium be adsorbed directly onto the adsorption holes 574 and 574a.

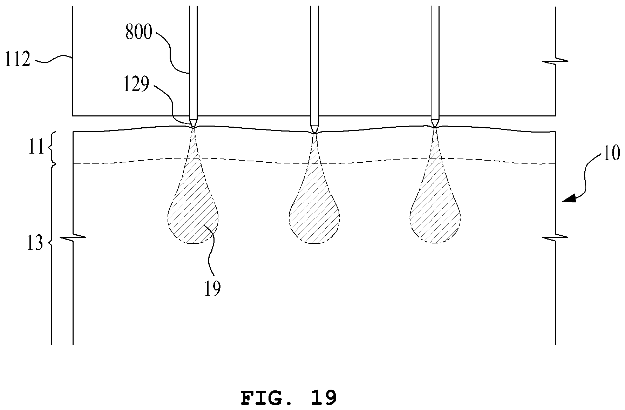

FIG. 19 is a cross-sectional view illustrating the results of the operation in which the Na-effect is achieved in an endosurgical operating device according to another embodiment of the present invention. In embodying the endosurgical operating device according to another embodiment of the present invention, the plurality of needles 121 in the endosurgical operating device according to another embodiment of the present invention are changed to a plurality of protrusions 800 provided with pointed tips whose end portions are ball-processed like the end of a ball-pointed pen, so that the tissue surface or the epithelial surface within the human body is not penetrated even if a predetermined pressure is applied.

As illustrated in FIG. 19, it can be seen that the Na-effect was achieved in the same way even in a nonpenetrated-in situation, as in the situation in which a predetermined pressure is applied to the tissue surface or epithelial surface within the patient to form points of contact on the plurality of protrusions 800.

The principle thereof will be described as below. There are no blood vessels in the epithelial layer 11, whereas a number of blood vessels are distributed in the lamina propria 13, and blood within the blood vessel has properties of ionic electrolyte.

Such ionic electrolyte is vibrated by high-frequency (especially RF) current in the protrusions 800 adhered to the epithelial surface. Accordingly, larger amount of heat than the heat generated from the epithelial layer 11 is generated in the lamina propria 13. Therefore, the operator may control the level and range of heat generation in the zone of the lamina propria 13 by controlling the intensity of RF current.

Meanwhile, except that a plurality of needles 300 are substituted to a plurality of protrusions 800, the operation condition of the Na-effect according to the sixth embodiment of the present invention in FIG. 19, and the operation condition of the Na-effect in FIG. 6 described above are identical.

In addition, except that the plurality of needles 121 are substituted to a plurality of protrusions 800, the endosurgical operating device and its structure and operation principle according to the first to fifth embodiments described above may equally applied to the endosurgical operating device according to the sixth embodiment of the present invention

The embodiments of the present invention described above may be applied not only for the use of the human body but also to the field of veterinary science.

Meanwhile, in embodying the present invention, an endoscopic device including the endoscope 1 may have a separate camera with a hole for camera photography, and optical cable necessary for camera photography may be embedded in any one of the plurality of needles 121.

In addition, a wireless communication module may be installed in the endoscopic device including the endoscope 1, so that the collected data may be sent out and remote operation may be conducted through the data transmitted from outside.

Meanwhile, pincers may be provided around the plurality of needles 121 for the case that adsorption of the endothelium adsorption means 170 of the present invention is not easy, so that the tissue of the operation portion is fixed with the pincers.

Meanwhile, additional insert tubes may be installed in the endoscopic device including the endoscope 1 according to the present invention so as to put in a knife, scissors, pincers, camera, foreign substances or smoke suction device for operation. Further, a tube may be formed in the needle 121 to insert laser fiber through it, so that the internal tissue may be irradiated with a laser beam.

In addition, the endoscopic device including the endoscope 1 according to the present invention may be provided additionally with a device whereby the tissue surface adsorbed through the adsorption means 170 is cooled to lower the epithelium surface temperature.

While the present invention has been described with reference to the preferred embodiments and modified examples, it will be understood by those skilled in the related art that various modifications and variations may be made therein without departing from the scope of the present invention as defined by the appended claims.

[Industrial Applicability]

The present invention has industrial applicability acknowledged in the field of medical appliances.

**Claims**

1. An endosurgical operating device 100 comprising:

an operation tube 111 configured to be installed in an insertion part 5 of an endoscope 1 to be inserted in a body;
an operation plate 122 which is installed in the operation tube 111 and includes, at least one needle 121 provided with a pointed tip or at least one protrusion 800 provided with a pointed tip;
a driving unit 165 configured to drive the operation plate 122 so that, at least one of the needles or the pointed tip of at least one protrusion 800, passes through an end portion of the operation tube 111, and is inserted into an internal tissue surface or an epithelium surface in the body; and
a power supply unit 140 which is electrically connected to, the at least one needle 121 or the at least one protrusion 800; and
an adsorption means 170 which is provided in the end portion of the operation tube 111,
wherein the adsorption means 170 has an adsorption means body 171 in which an air flow space 172 is formed;
an air flow means creating an air flow from an inlet to an outlet of the air flow space,
at least one adsorption hole 174, which communicates with the air flow space 172, is formed in the adsorption means body 171,
a diameter of the adsorption hole 174 is bigger than a cross-section of the needle 121 perpendicular to a longitudinal direction of the needle 121, and wherein the flow of air generated in the air flow space 172 by operation of the air flow means decreases the pressure in the air flow space 172 to adhere the tissue surface or epithelium surface to the adsorption means and to absorb substances discharged from the tissue surface or the epithelium surface into the endosurgical operating device through the adsorption hole, when the at least one needle 121 is inserted into the at least one adsorption hole 174 and the internal tissue surface or the epithelium surface in the body,
the endosurgical operating device further comprises a filtration means which filters the absorbed substances.

2. The endosurgical operating device according to claim 1, wherein at least one of the adsorption means 170 includes a thermal and electrical insulation member.

3. The endosurgical operating device according to claim 1, further comprising a temperature sensor configured to detect a temperature of the adsorption means 170.

4. The endosurgical operating device according to claim 1, wherein the at least one needle 121 has a hollow portion formed therein, and comprises a drug container connected to the hollow portion.

5. The endosurgical operating device according to claim 1, further comprising a surface temperature control means 499 configured to control the temperature of an operation portion in contact with the tissue surface or the epithelium surface.

6. The endosurgical operating device according to claim 1, wherein

the pointed tip is configured to receive a high-frequency current applied thereto, and
the pointed tip has a bipolar electrode system.

7. The endosurgical operating device according to claim 1, wherein at least one of the pointed tips has a polarity alternating with the polarity of a neighboring pointed tip.

8. The endosurgical operating device according to claim 1, further comprising a control unit 151 configured to control the driving unit 165 so that the at least one needle is inserted into the tissue surface or the epithelium surface in a preset depth.

9. An endoscopic device comprising: the endosurgical operating device according to claim 1.


**Patentansprüche**

1. Endochirurgische Operationsvorrichtung 100, umfassend:

   einen Operationsschlauch 111, der dazu konfiguriert ist, in einen Einführungsteil 5 eines in einen Körper einzuführenden Endoskops 1 installiert zu werden;
   eine Operationsplatte 122, die in den Operationsschlauch 111 installiert wird und mindestens eine Nadel 121, die mit einer scharfen Spitze versehen ist, oder mindestens einen Überstand 800, der mit einer scharfen Spitze versehen ist, beinhaltet;
   eine Antriebseinheit 165, die dazu konfiguriert ist, die Operationsplatte 122 anzutreiben, so dass mindestens eine von den Nadeln oder der scharfen Spitze von mindestens einem Überstand 800 durch einen Endabschnitt des Operationsschlauchs 111 hindurchtritt und in eine interne Gewebeoberfläche oder eine Epitheloberfläche in dem Körper eingeführt wird; und
   eine Energieversorgungseinheit 140, die mit der mindestens einen Nadel 121 oder dem mindestens einen Überstand 800 elektrisch verbunden ist; und
   ein Adsorptionsmittel 170, das in dem Endabschnitt des Operationsschlauchs 111 vorgesehen ist,
   wobei das Adsorptionsmittel 170 einen Adsorptionsmittelkörper 171 aufweist, in dem ein Luftstromraum 172 ausgebildet ist; wobei ein Luftstrommittel einen Luftstrom von einem Einlass zu einem Auslass des Luftstromraums erzeugt,
   wobei mindestens eine Adsorptionsöffnung 174, die mit dem Luftstromraum 172 kommuniziert, in dem Adsorptionsmittelkörper 171 ausgebildet ist,
   wobei ein Durchmesser der Adsorptionsöffnung 174 größer als ein Querschnitt der Nadel 121 senkrecht zu einer Längsrichtung der Nadel 121 ist
   und wobei der Luftstrom, der in dem Luftstromraum 172 durch Betrieb des Luftstrommittels erzeugt wird, den Druck in dem Luftstromraum 172 senkt, um die Gewebeoberfläche oder Epitheloberfläche an das Adsorptionsmittel zu haften und Substanzen, die von der Gewebeoberfläche oder der Epitheloberfläche abgesondert werden, in die endochirurgische Operationsvorrichtung durch die Adsorptionsöffnung zu absorbieren, wenn die mindestens eine Nadel 121 in die mindestens eine Adsorptionsöffnung 174 und die interne Gewebeoberfläche oder die Epitheloberfläche in dem Körper eingeführt wird,
   wobei die endochirurgische Operationsvorrichtung weiterhin ein Filtrationsmittel umfasst, das die absorbierten Substanzen filtert.

2. Endochirurgische Operationsvorrichtung nach Anspruch 1, wobei mindestens eines der Adsorptionsmittel 170 ein Wärme- und Elektroisolierelement beinhaltet.

3. Endochirurgische Operationsvorrichtung nach Anspruch 1, weiterhin umfassend einen Temperatursensor, der dazu konfiguriert ist, eine Temperatur des Adsorptionsmittels 170 zu erfassen.

4. Endochirurgische Operationsvorrichtung nach Anspruch 1, wobei die mindestens eine Nadel 121 einen darin ausgebildeten hohlen Abschnitt aufweist und einen Wirkstoffbehälter umfasst, der mit dem hohlen Abschnitt verbunden ist.

5. Endochirurgische Operationsvorrichtung nach Anspruch 1, weiterhin umfassend ein Oberflächentemperaturregelmittel 499, das dazu konfiguriert ist, die Temperatur eines Operationsabschnitts in Kontakt mit der Gewebeoberfläche oder der Epitheloberfläche zu regeln.

**6.** Endochirurgische Operationsvorrichtung nach Anspruch 1, wobei

die scharfe Spitze dazu konfiguriert ist, einen daran angelegten Hochfrequenzstrom aufzunehmen, und
die scharfe Spitze ein bipolares Elektrodensystem aufweist.

**7.** Endochirurgische Operationsvorrichtung nach Anspruch 1, wobei mindestens eine von den scharfen Spitzen eine Polarität aufweist, die mit der Polarität einer benachbarten scharfen Spitze alterniert.

**8.** Endochirurgische Operationsvorrichtung nach Anspruch 1, weiterhin umfassend eine Steuereinheit 151, die dazu konfiguriert ist, die Antriebseinheit 165 zu steuern, so dass die mindestens eine Nadel in die Gewebeoberfläche oder die Epitheloberfläche in einer voreingestellten Tiefe eingeführt wird.

**9.** Endoskopische Vorrichtung, umfassend: die endochirurgische Operationsvorrichtung nach Anspruch 1.

**Revendications**

**1.** Dispositif d'opération endochirurgicale 100 comprenant :

un tube d'opération 111 conçu pour être installé dans une pièce d'introduction 5 d'un endoscope 1 à introduire dans un corps ;
une plaque d'opération 122 qui est installée dans le tube d'opération 111 et qui comporte au moins une aiguille 121 pourvue d'un bout pointu ou au moins une partie saillante 800 pourvue d'un bout pointu ;
une unité d'entraînement 165 conçue pour entraîner la plaque d'opération 122 de sorte qu'au moins l'une des aiguilles ou le bout pointu d'au moins une partie saillante 800 passe dans une partie d'extrémité du tube d'opération 111 et est introduit dans une surface de tissu interne ou une surface d'épithélium dans le corps ; et
une unité d'alimentation électrique 140 qui est électriquement connectée à l'au moins une aiguille 121 ou l'au moins une partie saillante 800 ; et
un moyen d'adsorption 170 qui est disposé dans la partie d'extrémité du tube d'opération 111,
dans lequel le moyen d'adsorption 170 a un corps de moyen d'adsorption 171 dans lequel un espace de circulation d'air 172 est formé ; un moyen de circulation d'air créant une circulation d'air d'une entrée vers une sortie de l'espace de circulation d'air,
au moins un trou d'adsorption 174, qui communique avec l'espace de circulation d'air 172, est formé dans le corps de moyen d'adsorption 171,
un diamètre du trou d'adsorption 174 est plus grand qu'une section transversale de l'aiguille 121 perpendiculaire à une direction longitudinale de l'aiguille 121,
et dans lequel la circulation d'air produite dans l'espace de circulation d'air 172 par le fonctionnement du moyen de circulation d'air diminue la pression dans l'espace de circulation d'air 172 pour faire adhérer la surface de tissu ou la surface d'épithélium au moyen d'adsorption et pour absorber des substances évacuées à partir de la surface de tissu ou de la surface d'épithélium dans le dispositif d'opération endochirurgicale par le trou d'adsorption, lorsque l'au moins une aiguille 121 est introduite dans l'au moins un trou d'adsorption 174 et la surface de tissu interne ou la surface d'épithélium dans le corps,
le dispositif d'opération endochirurgicale comprend en outre un moyen de filtration qui filtre les substances absorbées.

**2.** Dispositif d'opération endochirurgicale selon la revendication 1, dans lequel au moins l'un des moyens d'adsorption 170 comporte un élément d'isolation thermique et électrique.

**3.** Dispositif d'opération endochirurgicale selon la revendication 1, comprenant en outre une sonde de température conçue pour détecter une température du moyen d'adsorption 170.

**4.** Dispositif d'opération endochirurgicale selon la revendication 1, dans lequel une partie creuse est formée dans l'au moins une aiguille 121 et l'au moins une aiguille 121 comprend un récipient à médicament raccordé à la partie creuse.

**5.** Dispositif d'opération endochirurgicale selon la revendication 1, comprenant en outre un moyen de régulation de température de surface 499 conçu pour réguler la température d'une partie d'opération en contact avec la surface de tissu ou la surface d'épithélium.

6. Dispositif d'opération endochirurgicale selon la revendication 1, dans lequel
   le bout pointu est conçu pour recevoir un courant de haute fréquence qui lui est appliqué et
   le bout pointu a un système d'électrodes bipolaires.

7. Dispositif d'opération endochirurgicale selon la revendication 1, dans lequel au moins l'un des bouts pointus a une polarité alternant avec la polarité d'un bout pointu voisin.

8. Dispositif d'opération endochirurgicale selon la revendication 1, comprenant en outre une unité de commande 151 conçue pour commander l'unité d'entraînement 165 de sorte que l'au moins une aiguille est introduite dans la surface de tissu ou la surface d'épithélium à une profondeur préréglée.

9. Dispositif endoscopique comprenant : le dispositif d'opération endochirurgicale selon la revendication 1.

FIG. 1

FIG. 2

**FIG. 3**

**FIG. 4**

**FIG. 5**

FIG. 6

FIG. 7

FIG. 8

FIG. 9

FIG. 10

**FIG. 11**

FIG. 12

FIG. 13

FIG. 14

FIG. 15

FIG. 16

**FIG. 17**

FIG. 18

FIG. 19

**EP 2 893 894 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20060088504
- KR 20090131724
- US 2002120260 A
- US 2005107829 A
- WO 2010022275 A
- US 3640270 A
- WO 2008005477 A